(19) Europäisches Patentamt
European Patent Office
Office européen des brevets

(11) EP 0 926 242 A1

(12) # EUROPEAN PATENT APPLICATION
published in accordance with Art. 158(3) EPC

(43) Date of publication:
**30.06.1999 Bulletin 1999/26**

(21) Application number: **97946814.7**

(22) Date of filing: **02.12.1997**

(51) Int. Cl.[6]: **C12P 17/18**, C07D 513/18, A61K 31/535
// (C12P17/18, C12R1:645)

(86) International application number:
**PCT/JP97/04404**

(87) International publication number:
**WO 98/24926 (11.06.1998 Gazette 1998/23)**

(84) Designated Contracting States:
**DE FR GB**

(30) Priority: **02.12.1996 JP 32137496**
**02.12.1996 JP 32137596**
**04.07.1997 JP 17940497**

(71) Applicant: **Ajinomoto Co., Inc.**
**Tokyo 104-0031 (JP)**

(72) Inventors:
- **MUKAI, Chika,**
**Central Research Laboratories**
**Kawasaki-shi, Kanagawa 210 (JP)**
- **OHSUMI, Koji,**
**Central Research Laboratories**
**Kawasaki-shi, Kanagawa 210 (JP)**
- **NAKAGAWA, Ryusuke,**
**Central Research Laboratories**
**Kawasaki-shi, Kanagawa 210 (JP)**
- **YOSHIMURA, Toshihiko,**
**Central Research Lab.**
**Kawasaki-shi, Kanagawa 210 (JP)**
- **KANAYAMA, Yuka,**
**Central Research Lab.**
**Kawasaki-shi, Kanagawa 210 (JP)**
- **OBAYASHI, Yoko,**
**Central Research Laboratories**
**Kawasaki-shi, Kanagawa 210 (JP)**
- **KAIDA, Kenichi,**
**Central Research Laboratories**
**Kawasaki-shi, Kanagawa 210 (JP)**
- **SUGA, Yasuyo,**
**Life Science Laboratories**
**Yokohama-shi, Kanagawa 210 (JP)**
- **MURATA, Masahiro,**
**Central Research Laboratories**
**Kawasaki-shi, Kanagawa 210 (JP)**

(74) Representative:
**Nicholls, Kathryn Margaret et al**
**MEWBURN ELLIS**
**York House**
**23 Kingsway**
**London WC2B 6HP (GB)**

(54) **GLIOTOXIN DERIVATIVES AND ANTICANCER AGENT COMPRISING THE SAME**

(57) Acetylgliotoxin and an anticancer agent comprising a gliotoxin derivative represented by formula (I) as the active ingredient. These gliotoxin compounds have excellent efficacy against solid cancer for which the conventional agents have only unsatisfactory efficacy.

EP 0 926 242 A1

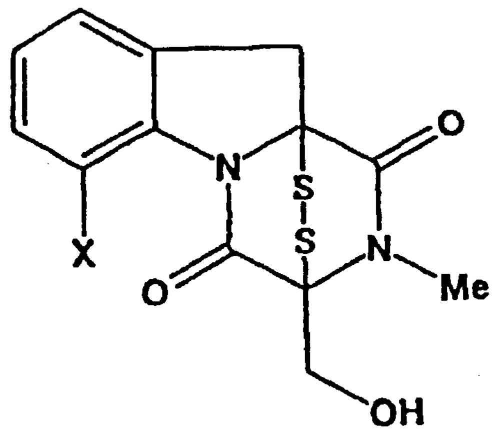

(I)

## Description

Technical Field

This invention relates to a gliotoxin derivative having an activity of inhibiting tumor cell multiplication and a anticancer agent containing it as an active principle.

Background Art

Heretofore, various anticancer agents for carcinoma have been developed, but a satisfactory anticancer agent has not been developed yet. Anticancer agents presently used in clinical field (Adriamycin, Cisplatin, 5-FU, Kanptotecin etc.) exhibit remarkable effects upon cancer species having good multiplication, such as leakemia cells. However, carcinomas which require injection of anticancer agent are inferior in multiplication, and the present situation is that these existing anticancer agents cannot exhibit their effects sufficiently. This is one reason for not exhibiting the existing anticancer agents sufficiently their effects on carcinomas in medical treatment.

An object of the invention is to provide an anticancer agent having cytotoxity without multiplication dependency and apoptosis-inducing ability, and being effective on carcinoma.

Desclosure of Invention

The inventors investigated in order to achieve the above object, and found that culture solution of a fungus (Dichotomomyces cejpii) has strong cytotoxity without multiplication dependency and apoptosis-inducing ability. Then, they separated the component, and identified to be acetylgliotoxin.

Acetylgliotoxin has the following formula.

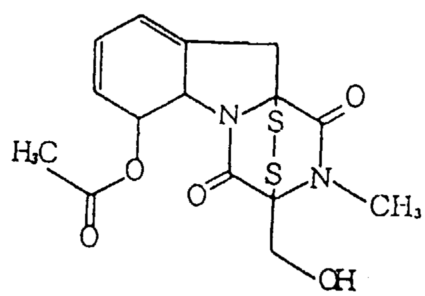

Then, they investigated physiological properties of acetylgliotoxin, and found that acetylgliotoxin has (1) cytotoxity without multiplication dependency, (2) apoptosis (cell death) -inducing ability, and exhibits (3) sufficient cytotoxity in a three dimensional culture using clinically extracted cancer cells, (4) remarkable growth inhibition against transplantation carcinomas (human carcinoma, mouse carcinoma) in a wide dose range in evaluation in vivo tests.

The acetylgliotoxin was already reported by J.R. Johnson (J. Amer. Chem. Soc., 75, 2110-2112 (1953)), and accordingly, is a known compound. However, it has not been known at all up to now that acetylgliotoxin exhibits anticancer effects.

Furthermore, it is also not known a means for mass production of acetylgliotoxin. Based upon the above findings, the inventors synthesized various compounds from the acetylgliotoxin, and investigated their anticancer activity.

As a result, they found that the gliotoxin derivatives represented by the following formula also exhibit great anticancer activity against carcinoma.

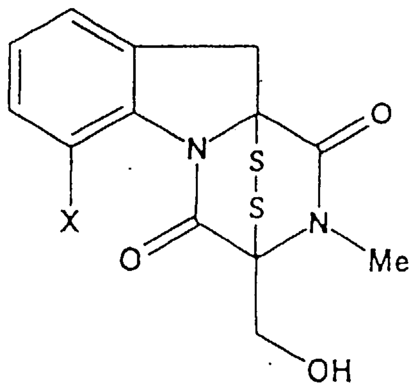

(I)

[In the above formula, X represents hydrogen atom, hydroxyl group, cyanomethyloxy group, an alkoxy group having a number of carbon atoms of 1 to 3, and acyloxy group having a number of carbon atoms of 2 to 3 or methoxycarbonylmethyloxy group.]

Among the compounds of the above formula, although the compound wherein X is hydroxyl group is known (J. Chem. Soc. (c), 1799 (1966)), the others are novel ones. It has not been known at all up to now that these compounds have anticancer activity.

That is, the present invention provides an anticancer agent comprising acetylgliotoxin or a glyotoxin derivative represented by the above formula as an active principle.

Brief Description of Drawings

Figure 1 shows $^{1}H$ nuclear magnetic resonance spectra of the acetylgliotoxin obtained in the invention.
Figure 2 shows $^{13}C$ nuclear magnetic resonance spectra of the acetylgliotoxin obtained in the invention.
Figure 3 is a graph indicating the results of measuring the time course of the weight of tumor while injecting the acetylgliotoxin obtained in the example of the invention into a mouse suffering from carcinoma of the colon.
Figure 4 is a graph indicating the results of measuring the time couse of IR while injecting the acetylgliotoxin obtained in the example of the invention into a mouse suffering from carcinoma of the colon.
Figure 5 is a graph indicating the results of measuring the time course of the weight of carcass while injecting the acetylgliotoxin obtained in the example of the invention into a mouse suffering from carcinoma of the colon.

Best Mode for Carring Out the Invention

The gliotoxin derivatives of the invention can be synthesized from acetylgliotoxin, and acetylgliotoxin can be mass-produced by culturing a microorganism capable of producing it. An illustrative of the microorganisms having acetylgliotoxin-producing ability is Dichotomomyces cejpii AJ 117330 (FERM BP-5738). The microorganism was obtained from the soil collected in Kawasaki-shi, Kanagawa, as follows: 1 g of the soil was heat-treated with moistening at 55 °C for 10 minutes, and applied by the dilution plating method over the whole surface of the cornmeal agar medium (antibiotic was added, 50 mg/L chloramphenicol, 15 mg/L rose bengal). Cultivation was carried out at 25 °C for 5 days, and the strain of the invention was isolated from the colonies appeared on the medium.

Mycological properties of AJ 117330 (FERM BP-5738) strain so isoloted are described below.

(a) Growth Form on Various Media

Growth on the malt extract agar medium is good, and the colony diameter at 25 °C for 7 days is 52 mm. The surface of the colony is white fluffy, and the reverse is light yellow. Production of dye and oil drop is not recognized in the medium. Hypha is loose wool-shaped, and cleistothecium is formed over the surface.

Growth on the potato-glucose agar medium is good, and the colony diameter at 25 °C for 7 days is 58 mm. The surface of the colony is white fluffy, and the reverse is light yellow. Production of dye and oil drop is not recognized in the medium. Hypha is loose wool-shaped, and cleistothecium is formed over the surface.

Growth on the cornmeal agar medium is good, and the colony diameter at 25 °C for 7 days is 47 mm. The surface of the colony is white fluffy, and the periphery is thin. The reverse is white. Production of dye and oil drop is not

recognized in the medium. Cleistothecium is formed.

(b) Morphological Properties

Cleistothecia scattered on the agar surface are sphere to subsphere having a diameter of 0.3 mm to 1 mm, and wrapped loosely by colorless mycelia. Ascus wrapped in ascocarp is colorless, and contains spores. The form of ascus is sphere to subsphere having a diameter of 8 to 12 µm. Ascospore is colorless, and its form is sphere on the front, lens on the side, and has 2 equatorial peripheries. The size is about 4 µm (not containing equatorial peripheries).

Conidium generation is formed of aleurioconidium. Branching system is irregular, and generally dichotomy. Conidium is oval, and the size is (6 to 10) × (5 to 8) µm.

Growth temperature is 10 to 40 °C, and optimum growth temperature is 25 to 35 °C.

Growth pH is 3 to 10, and optimum pH is 5 to 7.

Based on the above mycological properies, the strain was identified as Ascomycotina, Plectomycetes, Dichotomomyces cejpii, according to "Kinrui Zukan" (Autters: 6 persons, Shunichi Udagawa, Keisuke Tsubaki and others, Kodan-sha (Japan) 1978). This strain has been deposited in National Instituted of Bioscience and Human Technology, Agency of Industrial Science and Technology (1-3, Higashi 1-chome, Tsukuba-shi, Ibaraki-ken, Japan) since November 6, 1996, based on Budapest Treaty on the International Recognition of the Deposit, and an accession number of FERM BP-5738 was added.

The medium for culturing a microorganism belonging to genus Dichotomomyces and having acetylgliotoxin-producing ability may be a medium capable of culturing a mould, and a medium, which contains a carbon source, nitrogen source, inorganic salts, organic nutrients, etc., is usable, irrespective of solid medium or liquid medium. As a component of the medium, vegetable juice is effective, and it is preferable to add in an amount of about 50 g/L to 200 g/L.

Culturing can be carried out at about 10 to 40 °C, preferably 25 to 35 °C for about 5 to 14 days, usually 5 to 10 days under aerobic conditions.

Upon collecting acetylgliotoxin from the culture matter, it is preferable to extract the acetylgliotoxin remaining in the fungus cells by adding a hydrophilic organic solvent, such as acetone, methanol or ethanol, as a preliminary treatment. A suitable amount to be added is about 1/4 to 1/1 as a volume ratio against the culture matter. The above amount is in the case that the culture matter is a culture solution, and when the culture solution is preliminarily concentrated by vacuum concentration or the like, the amount is against the concentrate. In the case that the remaining amount in the cells is small, this treatment is not necessary.

As a means for recovering acetylgliotoxin from the culture matter, there are solvent extraction and adsorption separation. In the case of the solvent extraction, an organic solvent which can dissolve acetylgliotoxin is added to the culture matter. A suitable amount to be added is about 1/4 to 1/1, usually about 1/2 to 1/1 as a volume ratio against the culture matter. The organic solvent may be water-miscible ones, such as acetone, $C_1$ to $C_4$ lower alcohols and dimethyl sulfoxide, or layer splitting ones from water, such as chloroform, ethyl acetate and toluene. In the case of using a water-miscible organic solvent, suspended matters, such as cells are separated by centrifugation, filtration or the like. In the case of using a layer splitting organic solvent from water, the water layer is separated, and the supernatant or the organic solvent layer is collected and subjected to purification.

In the case of the adsorption separation, an adsorbent, such as a synthetic adsorbent (e.g. "Diaion HP 20", "Sepabeads SP 207", Mitsubishi Chemical), is allowed to contact with the culture solution to recover acetylgliotoxin by adsorption.

Purification may be carried out by a combination of adsorption separation by a silica gel column, liquid chromatography, solvent extraction and so on.

The gliotoxin derivatives of the invention are represented by the aforementioned general formula (I) wherein X is hydrogen, hydroxyl group, an alkoxy group haivng a number of carbon atoms of 1 to 3 or an acyloxy group having a number of carbon atoms of 2 to 3.

Illustrative of the alkoxy groups having a number of carbon atoms are methoxy group, ethoxy group and propyloxy group. Illustrative of the acyloxy group having a number of carbon atoms of 2 to 3 are acetyl group and propionyl group.

Illustrative of the gliotoxin derivatives represented by the general formula (I) are:

X : hydrogen atom
(3S, 10aR)-2,3-dihydro-3-(hydroxymethyl)-2-methyl-10H-3,10a-epidithiopyrazino[1,2-a]indole-1,4-dione
X : hydroxyl group
(3S, 10aR)-6-hydroxy-2,3-dihydro-3-hydroxymethyl-2-methyl-10H-3,10a-epidithiopyrazino[1,2-a]indole-1,4-dione
X : cyanomethyloxy group
(3S, 10aR)-6-(cyanomethoxy)-2,3-dihydro-3-hydroxymethyl)-2-methyl-10H-3,10a-a-epidithiopyrazino[1,2-a]indole-1,4-dione

X : methoxy group
(3S, 10aR)-6-methoxy-2,3-dihydro-3-(hydroxymethyl)-2-methyl-10H-3,10a-epidithiopyrazino[1,2-a]indole-1,4-dione
X : ethoxy group
(3S, 10aR)-6-ethoxy-2,3-dihydro-3-hydroxymethyl-2-methyl-10H-3,10a-epidithiopyrazino[1,2-a]indole-1,4-dione
X : propoxy group
(3S, 10aR)-6-propyloxy-2,3-dihydro-3-(hydroxymethyl)-2-methyl-10H-3,10a-epidithiopyrazino[1,2-a]indole-1,4-dione
X : acetoxy group
(3S, 10aR)-6-acetoxy-2,3-dihydro-3-(hydroxymethyl)-2-methyl-10H-3,10a-epidithiopyrazino[1,2-a]indole-1,4-dione
X : propionyloxy group
(3S, 10aR)-6-propionyloxy-2,3-dihydro-3-(hydroxymethyl)-2-methyl-10H-3,10a-epidithiopyrazino[1,2-a]indole-1,4-dione
X : methoxycarbonylmethyloxy group
(3S, 10aR)-2,3-dihydro-3-(hydroxymethyl)-6-[ (methoxycarbonyl)methyloxy]-2-methyl-10H-3,10a-epidithiopyrazino[1,2-a]indole-1,4-dione

The compound of the invention represented by the general formula (I) can be synthesized, for example, according to the following manufacturing route.

That is, the compound (4) wherein X is hydrogen atom can be obtained by protecting disulfide bond of the compound (1) by para-anisaldehyde, converting the compound (1) to the compound (2), eliminating acetyl group of the compound (2) by methoxide to produce the compound (3), and then, eliminating the protective group by Lewis acid.

The route is expressed by formulas as follows:

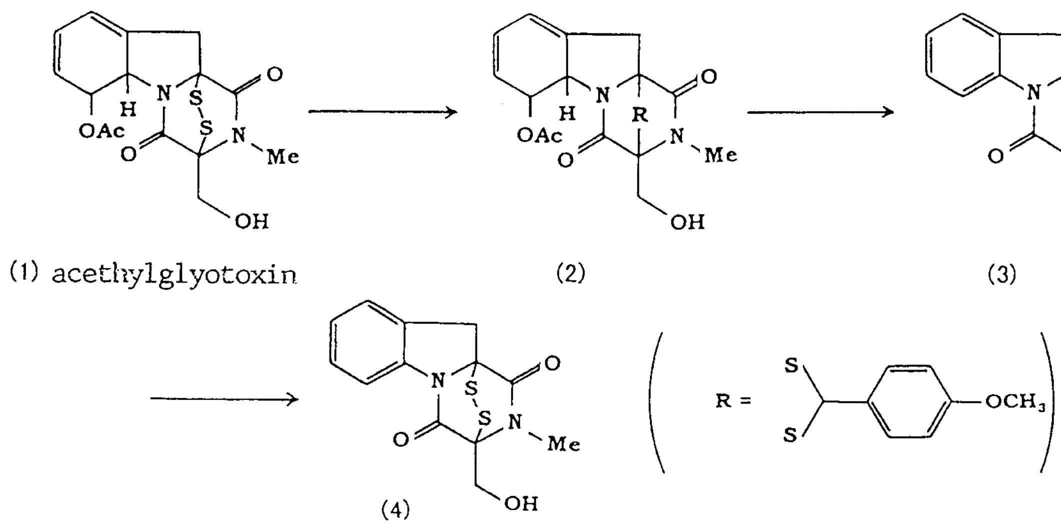

(1) acethylglyotoxin (2) (3)

(4)

The compound (G) wherein X is hydroxyl group can be obtained by allowing the compound (1) to react with tosyl acid, converting acetoxy group to hydroxyl group to obtaine the compound (5), and then, oxidizing it by chloranil. The above compound (5) is gliotoxin (Tetrahedron, 37, 2045 (1981), etc.), and can be synthesized according to a method described in the known reference.

The route is expressed by formulas as follows:

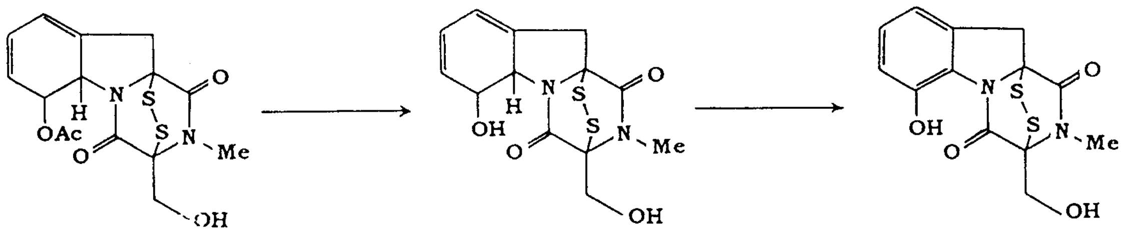

(1) acetyltlyotoxin (5) glyotoxin (6)

The compound (9) wherein X is alkoxy group can be obtained by alkylating the compound (7) by all alkyl halide and potassium carbonate to produce the compound (8), and then, eliminating the protective group by Lewis acid. Illustrative of the alkyl halides are methyl iodide, butyl bromide, propyl bromide, bromoacetonitrile, bromomethyl acetate and the like.

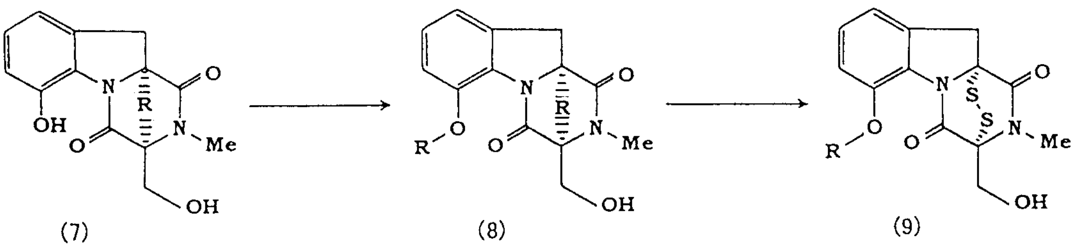

(7) (8) (9)

The compound (13) wherein X is acetoxy group can be obtained by converting acetoxy group of the compound (2) to hydroxyl group by hydrochloric acid, oxidizing it by orthochloranil to produce the compound (11), acetylated by acetic anhydride, and then, eliminating the protective group by Lewis acid.

The route is expressed by formulas as follows:

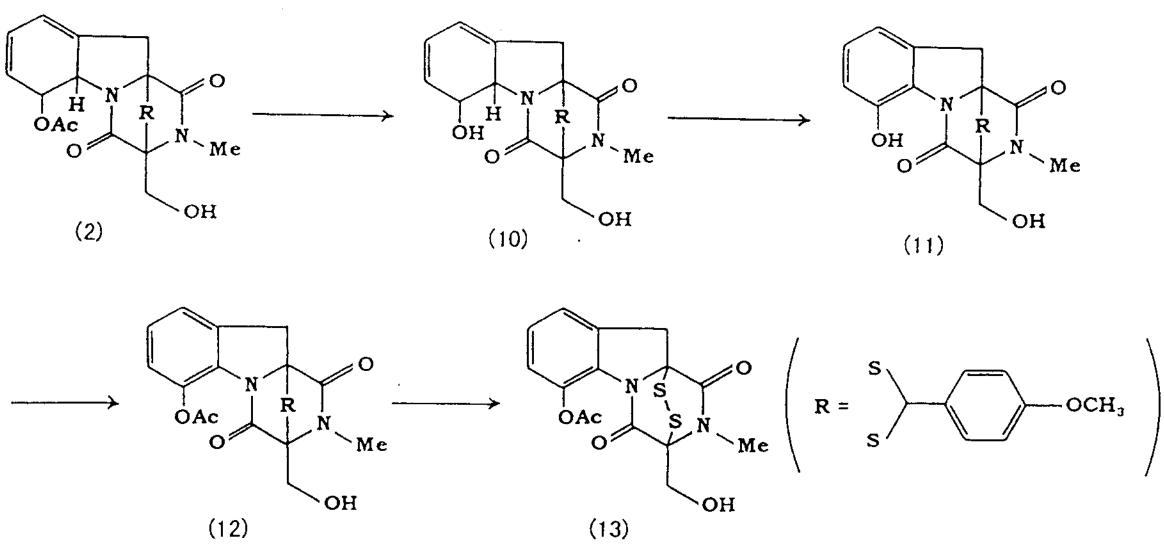

(2) (10) (11)

(12) (13)

In addition, the compound wherein X is propionyloxy group can be synthesized similarly by propionylating the compound (8).

The gliotoxin derivatives manufactured by the above method can be separated from the reaction mixture and purified easily by conventional isolation, purification means, such as solvent extraction, chromatography and crystallization, similar to the aforementioned acetylgtiotoxin.

In the case of using acetylgliotoxin or the gliotoxin derivative as anticancer agent, it is administered by oral administration or non-oral administration (intramuscular injection, subcutaneous injection, intravenous injection, suppository, etc.). A suitable dose is, in general, 1 to 9000 mg per one adult per 1 day and the administration may be divided into several times, once 1 to 3000 mg, although the dose varies according to the condition of a patient.

Furthermore, in the case of preparing the gliotoxin derivative of the invention as a medicine for oral administration, an excipient and optionally a bonding agent, a collapsing agent, a smoothing and glossing agent, a coloring agent, a taste-corrective smell-corrective agent and the like are added, and then, formed into tablets, coated tablets, granules, capsules or the like, according to the conventional manner.

The excipient is lactose, corn starch, white sugar, glucose, sorbit, crystalline cellulose or the like. The bonding agent is polyvinyl alcohol, polyvinyl ether, ethyl cellulose, methyl cellulose, gum arabic, tragacanth, gelatin, shellac, (hydroxypropyl cellulose, hydroxypropyl starch,) polyvinylpyrrolidone or the like. The collapsing agent is starch, agar, gelatin powder, crystalline cellulose, calcium carbonate, sodium hydrogen carbonate, calcium citrate, dextran, pectin or the like. The smoothing and glossing agent is magnesium stearate, talc, polyethylene glycol, silica, hardened vegetable oil or the like. As the coloring agent, there are those allowed as the additives of medicines. The taste-corrective smell-corrective agent is cocoa powder, menthol, aromatic acid, peppermint oil, refined Borneo camphor, cinnamon powder or the like. The tablets and granules may be provided with sugar coating, gelatin coating or other coatings.

In the case of preparing medicine for injection, a pH adjuster, a buffer, a stabilizer, a preservative or the like are optionally added, and made into medicines for subcutaneous, intramuscular or intravenous injection, according to the conventional manner.

The gliotoxin derivative of the invention can exhibit the effect of medicine sufficiently even in a form of a pharmacologically allowable acid adduct salt, the acid may be an inorganic acid, such as hydrochloric acid, sulfuric acid or phosphoric acid, or an organic acid, such as oxalic acid, fumaric acid, maleic acid, malic acid, citric acid, tartaric acid or glutamic acid.

Example 1

(1) Cultivation

Acetylgliotoxin can be obtained by culturing the aforementioned mould (Dichotomomyces cejpii) AJ 117330 (FERM BP-5738) strain in a culture medium, and collecting it from the culture solution. The cultivation of the mould was carried out as follows: The mould was inoculated on the malt extract agar medium, and cultured at 25 °C for 7 days. Agar pieces were cut off the malt extract agar medium, and inoculated into 100 mL of the medium for production (medium A) having the following composition placed in a roux-type flask (height of liquid surface: about 1 cm). Then, stationary culture was carried out at 25 °C for 8 days.

On the other hand, the above agar pieces cut off the malt extract agar medium were inoculated into 100 mL of the medium for production (medium A) placed in a 500 mL Erlenmeyer flask, and rotary culture was carried out at 25 °C for 6 days. 4 batches (400 mL) of the above culture solution were inoculated into 20 L of the medium A placed in a 20 L zar, and cultured at 25 °C at 250 rotations/min at an aeration rate of 1/2 vvm for 6 days.

[Composition of Medium A]

| | |
|---|---|
| Vegetable juice (non-salt, Kagome) | 100 g/L |
| Potato dextrose broth (Difco) | 12 g/L |
| Yeast extract (Difco) | 2 g/L |
| Soluble starch | 15 g/L |
| $KH_2PO_4$ | 1.0 g/L |
| $MgSO_4 \cdot 7H_2O$ | 0.5 g/L |
| $CaCl_2 \cdot 7H_2O$ | 0.5 g/L |
| 2- (N-Morpholino) ethanesulfonic acid | 2 g/L |

(2) Purification to Isolate Acetylgliotoxin

After cultivation by the above method was finished, an equal amount (3 L) of acetone was added to the 3 L culture solution, and extraction was carried out at room temperature for 30 minutes. Acetone was removed from the acetone-containing cultuer solution by an evaporator, and solvent extraction with ethyl acetate was carried out by a separating funnel. The solvent extract was dehydrated by sodium sulfate, and then, evaporated by an evaporator to dryness under reduced poressure to obtain 470 mg of the extract. Subsequently, the extract was adsorbed on a silica gel column, washed with toluene, and active fractions were eluted by toluene/ethyl acetate (8:2). The eluate was concentrated by an evaporator under reduced pressure to obtain 42 mg. The crude matters were subjected to high speed liquid chromatography ("Capsel-Pak $C_{18}$", Shiseido, solvent: water:acetonitrile = 72:28), and a pure active substance was obtained by isochromatic elution. The physicochemical properties of the active component were measured as follows:

| | |
|---|---|
| Solubility: | Easily soluble in chloroform and dimethyl sulfoxide, soluble in ethyl acetate, acetonitrile, toluene, methanol and acetone, slightly soluble in water. |
| Ultraviolet absorption spectra | end 266 nm (water: acetonitrile = 60 : 40) |
| Molecular weight: | 368 |
| Thin layer chromatography: | Absorbent; HPTLC Kieselgel 60 F 254 (MERK) |
| Developing solvent: | hexane : ethyl acetate = 6 : 4<br>R f value; 0.233 |
| High speed liquid chromatogaphy: | |
| Used apparatus; UV detector : | Waters 996 (Waters) |
| Pump: | HITACHI L—6200 (Hitachi Ltd.) |
| Column: | Reversed phase high speed liquid chromatography column for analysis (CAPCELL PAK C18 UG120 5 μ m 4.6 mm ∅ × 250 mm (Shiseido)<br>Solvent for elution; 30-100 % acetonitrile (linear grudient 20 minutes)<br>Flow rate; 0.8 mL/min<br>Retention time; about 10.5 minutes |

Chemical shift values (cf. Fig. 1) in $^1H$ nuclear magnetic resonance spectra

$^1H$—NMR ($CDCl_3$) δ :5.97—6.04 (3H, m), 5.57 (1H, d), 5.02 (1H, d), 4.31 (1H, dd), 4.19 (1H, dd), 3.74 (1H, d), 3.44 (1H, dd), 3.17 (3H, s), 2.96 (1H, d), 2.14 (3H, s)

FAB MASS : 369 ($MH^+$)

Chemical shift values (cf. Fig. 2) in $^{13}C$ nuclear magnetic resonance spectra (600 $MH_3$, $CDCL_3$)

Mass spectroscopy by a mass spectrometer and H-NMR measurement of the active component were carried out, and retrieval was carried out by an on-line system based on the data, and it was found that the active component is acetylgliotoxin.

(3) Measurement of Cytotoxity of Acetylgliotoxin

Against Various Cancer Cells and Judgement of Apoptosis

Antitumor activity of acetylgliotoxin against various cancer cells was measured by the MTT method. Various cancer cells were adjusted to $4.0 \times 10^4$ cells/mL by using a medium (hereinafter referred to as medium A) composed of RPMI 1640 medium (GIBCO) containing 10% fetal calf serum, and each 0.1 mL of the cancer cells was pipetted into each well of a 96 well microtiter plate. The plate was incubated in a carbon dioxide gas incubator at 37 °C for 20 hours, and each 0.025 mL of samples (test compounds) suitably diluted with the medium A was added. Then, incubation was further carried out in the carbon dioxide gas incubator at 37 °C for 72 hours. Each 0.01 mL of the MTT solution adjusted to 5 mg/mL by using Dulbecco's PBS (-) (Nissui) was added thereto, and incubated in the carbon dioxide gas incubator at 37 °C for 3 hours. Subsequently, each 0.05 mL of 0.01 N HCl/20 % SDS was added. Produced crystals were dissolved, and absorbance at 570 nm was measured by a microplate reader. By comparing the absorbance of the cells treated with the sample in the known concentration with that of non-treated cells, the sample concentration which inhibits multiplication of the cells at the rate of 50% ($IC_{50}$) was calculated. The results are shown in Table 1. Apoptosis was determined by incubating the plate in the carbon dioxide gas incubator at 37 °C for 20 hours, adding each 0.025 mL of samples (test compounds) suitably diluted with the medium A, incubating in the carbon dioxide gas incubator at 37 °C for 72 hours, according to the above method, adding nucleus-staining fluorescent dye DAPI (4,6 - diaminodino-2-henylindole) to each well so as to become 0.5 ng/mL, and after leaving at room temperature for 10 minutes, confirming agglomeration of chromosomes by using a fluorescence microscope.

[Cells Used for the Measurement of Cytotoxity]

Human leukemia cells (K 562 : Dainippon Seiyaku), human carcinoma cells of the colon (HCT-15, HCT-116, SW946: Dainippon Seiyaku), human leukemia cells (U 937: Dainippon Seiyaku), human lung cancer cells (LX-1: Biseibutsu Kagaku Kenkyu-sho), mouse leukemia cells (P 388 : Dainippon Seiyaku), mouse carcinoma cells of the colon (colon-26 : Gan Kenkyu-kai, Gan Kenkyu-sho)

Table 1

| Type of Cells | I $C_{50}$ (ng/mL) |
|---|---|
| HCT-15 (human carcinoma of the colon) | 39 |
| HCT-116 (human carcinoma of the colon) | 180 |
| SW948 (human carcinoma of the colon) | 190 |
| LX-1 (human lung cancer ) | 74 |
| U937 (human leukemia ) | 14 |
| P388 (mouse leukemia ) | 5.8 |
| colon-26 (mouse carcinoma of the colon ) | 76 |

(4) Tertiary Culture Using Clinically Extracted Tumor

Tertiary cultures were carried out using a human carcinoma of the colon tissue in stage 2b. The tertiary cultures were carried out according to the reported method (Int.J.Cancer Res., 82, 607-612 (1991), Gan to Kagakuryoho, 18, 239-243 (1991), Gan to Kagakuryoho, 19, 743-748 (1992)). The extracted tissue of carninoma of the colon was cut into square pieces having a side of about 2 mm, and incubated in a RPMI- 1640 medium containing 10 % fetal calf serum under 5 % $CO_2$ at 37 °C for 3 days. Existing anticancer agents or acetylgliotoxin were diluted and added, and further incubated for 3 days. Then, the rate of killed cells was determined by the MTT method. The tertiary cultures using the test human cancer were carried out as follows: Human carcinoma of the colon cell strain HCT-116 was transplanted into the subcutaneous tissue of a nude mouse (Balb nu/nu) to induce tumor. The cancer tissue was extracted from the tumor-induced portion, and cut into square pieces having a side of 2 mm. The evaluation of the existing anticancer agents and acetylyliotoxin was earried out according to the aforementioned method. The results are shown in Table 2.

Tertiary Culture Using Test Human Cancer

Table 2

| | conc. (ug/mL) | I. R. (%) |
|---|---|---|
| A D R | 6 | 0 |
| C D D P | 25 | 6 |
| M M C | 15 | 34 |
| T A X O L | 100 | 19 |
| Vinblastine | 100 | 20 |
| 5-F U | 500 | 38 |
| Gliotoxin | 25 | 66 |
| | 12.5 | 28 |

Table 2 (continued)

| | conc. (ug/mL) | I. R. (%) |
|---|---|---|
| Acetyl-Gliotoxin | 25 | 67 |
| | 12.5 | 56 |
| | 6.25 | 43 |

(Abbreviations)

ADM : Adriamycin
CDDP : Cisplatin
MMC : Mitomycin C
Taxol : Taxol
Vinblastine : Vinblastine
5-FU : 5-Fluorouracil

(5) Anticancer effect in vivo against carcinomas

(A) Evaluation in vivo using mouse carcinoma of the colon COLON — 26

CDFI mice (female: 4 weeks old) were bought, and colon-26 subcultured in vivo in the subcutaneous tissue was taken out. The colon-26 was minced in a Petridish, and transplanted into the sulcutaneous tissue of CDFI mice in an amount of 10 mg/mouse by a trucker. The transplanted day was assigned day 0, and the size of the tumor and body weight were measured on day 7 and divided into groups by n=4. The size of tumor was calculated as tumor weight by the following formula.

$$\text{Tumor Weight (mg)} = \text{Tumor Volume (mm}^3) = 1/2 \times \text{Major Axis (mm)} \times \text{Minor Axis (mm)}^2$$

Administration was carried out from day 7 according to the following schedule. Dose was 0.2 mL/mouse, and injected into abdominal cavity. Acetylgliotoxin was dissolved in dimethyl sulfoxide (DMSO) in a concentration of 100 mg/mL, and adjusted to an object concentration (0.5 mg/kg, 1 mg/kg, 10 mg/kg, 20 mg/kg) by 5% Tween 80 (saline solution). In the evaluation of 0.6, 1 mg/kg, injection was carried out every day for 5 days, in the case of 10 mg/kg, injected twice on day 7 and day 8, and in the case of 20 mg/kg, injected once on day 7.

The anticancer effect was evaluated by the inhibition rate of tumor cell multiplication (IR calculated from the weight of tumor. IR was obtained by the following formula.

$$\text{IR (\%)} = (1\text{-Tumor Weight of Administration Group/Tumor Weight of Control Group})$$

IR in each dose is shown below.

(B) Evaluation in vivo using human carcinoma of the colon HCT-116

Balb/c nu/nu mice (female: 5 weeks old) were bought, and HCT- 116 subcultured in vivo in the subcutaneous tissue was taken out. The HCT-116 was minced in a Petridish, and transplanted into the sulcutaneous tissue of CDFI mice in an amount of 30 mg/mouse by a trucker. The transplanted day was assigned day 0, and the size of the tumor and body weight were measured on day 7 and divided into groups by n=2.

Acetylgliotoxin was dissolved in dimethyl sulfoxide (DMSO) in a concentration of 100 mg/mL, and adjusted to an object concentration (0.5 mg/kg, 1 mg/kg, 10 mg/kg, 20 mg/kg) by 5 % Tween 80 (saline solution). In the evaluation of 0.5, 1 mg/kg, injection was carried out every day for 5 days, in the case of 10 mg/kg, injected twice on day 7 and day8, and in the case of 20 mg/kg, injected once on day 7.

The anticancer effect was evaluated by the inhibition rate of tumor cell multiplication (IR calculated from the weight of tumor. IR was obtained by the following formula.

The results of Test Examples (A), (B) are shown in Tables 3, 4 and Figures 3 to 5. Table 3 shows 1R and body weight on day 2 1 in each dose of acetylgtiotoxin in the evaluation in vivo using mouse carcinoma of the colon COLON-26, and Table 4 shows IR and body weight on day 21 in each dose of acetylgliotoxin in the evaluation in vivo using

human carcinoma of the colon HCT-116, respectively.

Table 3

| | I.R. (%) | weight (g) |
|---|---|---|
| 0.5 mg/kg×5 i.p.(d7-11) | 28 | 24.7 |
| 1 mg/kg×5 i.p.(d7-11) | 19 | 24.9 |
| 10 mg/kg×2 i.p.(d7, 8) | 38 | 24.1 |
| 20 mg/kg× 1 i.p.(d7) | 34 | 25.2 |
| (control 24.7 g ) | | |

Table 4

| | I.R.(%) | weight (g) |
|---|---|---|
| 0.5 mg/kg×5 i.p.(d7-11) | 41 | 17.1 |
| 1 mg/kg×5 i.p.(d7-11) | 65 | 17.8 |
| 10 mg/kg×2 i.p.(d7, 8) | 65 | 15.8 |
| 20 mg/kg×1 i.p. (d7) | 28 | 15.9 |
| (control 17.5 g ) | | |

Figure 3 shows time course of tumor weight, Figure 4 shows time course of 1R, and Figure 5 shows time course of carcass weight (mouse body weight-tumor weight), respectively. In respective figures, (A) indicates the results of the evaluation in vivo using mouse carcinoma of the colon COLON-26, (B) indicates the results of the evaluation in vivo using human carcinoma of the colon HCT-116, and acetylgliotoxin exhibited great anticancer effects, i.e. IR 65 % at 1 mg/kg × 5 and IR 65 % at 10 g/kg × 2 on day 21 in Test Example (B). The effects continued up to day 42. Accordingly, the anticancer agent of the invention is very effective in terms of not only being excellent cytotoxity without multiplication dependency but also having a sufficient possibility for clinical treatment by the selection of dose.

Example 2

The mould Dichotomomyces cejpii AJ 117330 (FERM BP-5738) was cultured similar to Example 1. To 40 L of the culture supernatant, 4 kg of "Diaion HP-20" was added, and stirred at room temperature for 1 hour. Then, the resin was separated, and elution was carried out using 17 L of acetone. Acetone was evaporated by the concentration under reduced pressure to obtain an aqueous solution, and extraction was carried out using 4 L of ethyl acetate. The ethyl acetate was washed with saturated sodium chloride solution, dehydrated by anhydrous sodium sulfate, and concentrated under reduced pressure. The residue thus obtained was dissolved in acetonitrile, and concentrated to dryness with a small amount of silica gel. Then, it was subjected to silica gel chromatography, and fractions eluted by toluene: hexane (8:2 and 7:3) were collected. The fractions were concentrated under reduced pressure to obtain 5.6 g of crude substance. It was dissolved in methanol, and crystallized at 4 °C and -20 °C.

Example 3

Synthesis of the compound (4) wherein X is hydrogen atom: (3S, 10aR)—2,3-dihydro—3—(hydroxymethyl)—2—methyl—10H—3,10a—epidithiopyrazino[1,2—a]indole— 1,4—dione

Process 1

Synthesis of (3S, 5aS, 6S, 10aR)—6—acetoxy—2,3,5a,6— tetrahydro—3—(hydroxymethyl)—2—methyl—10H—3,10a— [epithio—(4—methoxyphenyl)methanothio]pyrazino[1,2—a] indole—1,4—dione

Acetylgliotoxin (471 mg, 1.28 mmol) was dissolved in a solvent mixture of 20 mL dichloromethane and 3 mL methanol, and 105 mg (2.78 mmol) sodium borohydride was added while cooling in an ice bath, followed by stirring for 30 minutes. After confirming the finish of reduction by TLC, 3.7 mL (30.4 mmol) p-anisaldehyde, 3.5 mL (28.4 mmol) boron trifluoride ethyl ether complex salt were added, and stirred at room temperature for 40 minutes. Saturated sodium bicarbonate aqueous solution was added, and extraction was carried out by dichloromethane. The organic layer was washed with saturated sodium chloride aqueous solution, and after drying by anhydrous sodium sulfate, the solvent was evaporated under reduced pressure. The residue obtained was purified by silica gel chromatography (100 % dichloromethane) to obtain the object material in a form of light yellow oil. (553 mg yield:88 %)

$^1$H—NMR ($CDCl_3$) δ :2.22 (3H, s), 2.66 (1H, dd, J=4.73, 10.7Hz) ,2.97 (1H, d, J=18.5 Hz) , 3.16 (3H, s) , 3.36 (1H, d, J=18.5Hz), 3.74 (1H, dd, J=10.7, 12.9Hz), 3.79 (3H, s), 4.48 (1H, dd, J=4.73, 12.9Hz), 5.17 (1H, d, J=14.7Hz), 5.56— 5.61 (1H, m), 5.95—6.06 (2H, m), 6.08 (1H, s), 6.18 (1H, d, J=14.7Hz), 6.85 (2H, d, J=8.85Hz), 7.50 (2H, d, J=8.85Hz)ESI MASS: 511.3 ($M^+$+$Na^+$)

Process 2

Syntesis of (3S, 10aR)—2,3—dihydro—3—(hydroxymethyl) —2—methyl—10H—3,10a—[epithio—(4—methoxyphenyl) methanothio]pyrazino[1,2—a]indole—1,4—dione

(3S, 5aS, 6S, 10aR)—6—acetoxy—2,3,5a,6—tetrahydro—3 —(hydroxymethyl)—2—methyl—10H—3,10a—[epithio—(4—methoxyphenyl)methanothio]pyrazino[1,2—a]indole—1,4 —dione (412 mg, 0.843 mmol) was dissolved in a solvent mixture of 36 mL dichloromethane and 4 mL methanol, and 175 μL sodium methaxide (28 % methanol solution) was added, followed by stirring at room temperature for 1 hour 20 minutes. Then, water was added, and adjusted to pH 7 by 2NHCl. Extraction was carried out using dichloromethane, and the organic layer was washed with saturated sodium chloride aqueous solution. Alter drying by anhydrous sodium sulfate, solvent was evaporated under redued pressure. The residue obtained was purified by silica gel chromatography (100 % dichloromethane) to obtain the object material in a form of light yellow solid.
(294 mg yield: 81%)

$^1$H—NMR($CDCl_3$) δ :2.69 (1H, dd, J=5.10, 9.90Hz), 3.26 (3H, s), 3.29 (1H, d, J=17.3Hz), 3.77 (3H, s), 3.90 (1H, dd, J=9.90, 12.8Hz), 3.97 (1H, d, J= 17.3Hz), 4.72 (1H, dd, J=5.10, 12.8Hz), 5.30 (1H, s), 6.81 (2H, d, J=8.85Hz), 7.23 (1H, dd, J=7.20, 8.10Hz), 7.28 (2H, d, J=8.85Hz), 7.32 (1H, d, J=7.20Hz), 7.37 (1H, dd, J=8.10, 8.10Hz), 8.20 (1H, d, J=8.10Hz)
ESI MASS:451.3 ($M^+$+$Na^+$)

Process 3

Synthesis of (3S, 10aR)—2,3—dihydro—3—(hydroxymethyl)— 2—methyl—10H—3,10a—epidithiopyrazino[1,2—a]indole—1,4—dione

(3S, 10aR)—2,3—dihydro—3—(hydroxymethyl)—2—methyl—10H—3,10a—[epithio—(4— methoxyphenyl)methanothio] pyrazino[1,2—a]indole—1,4—dione (204 mg, 0.476 mmol) was dissolved in 100 mL dichloromethane, and 110 mg (0.637 mmol) m-chloroperbenzoic acid was added while cooling in an ice bath. After stirring for 40 minutes, 39.3 mg (0.228 mmol) m-chloroperbenzoic acid was further added, and then, stirred for 25 minutes. 130 μL (1.77 mmol) dimethylsulfide and 290 μL perchloric acid-methanol solution (1:5) were added, and stirred at room temperature for 20 hours 30 minutes. Then, saturated sodium bicarbonate aqueous solution was added, and extraction was carried out using dichloromethane. The organic layer was washed with saturated sodium chloride aqueous solution. After drying by anhydrous sodium sulfate, solvent was evaporated under redued pressure. The residue obtained was purified by silica gel chromatography (100 % dichloromethane), and recrystallized from methanol to obtain the object material as light yellow crystals (29.8 mg, yield : 20%).

$^1H$—NMR($CDCl_3$) δ :3.26 (3H,s), 3.34 (1H, d, J=18.8Hz), 3.48 (1H, dd, J=5.65, 9.80Hz), 4.29 (1H, dd, J=9.80, 12.7Hz), 4.32 (1H, d, J=18.8Hz), 4.48 (1H, dd, J=5.65, 12.7Hz), 7.22 (1H, dd, J=7.50, 7.50Hz), 7.33-7.38 (2H, m), 7.94 (1H, d, J=7.50Hz)
High resolution mass spectra: $C_{13}H_{13}N_2O_3S_2$ ($MH^+$)
Calcd.:m/z 309.0368
Measured.:m/z 309.0366

Example 4

Synthesis of the compound (6) wherein X is hydroxyl group: (3 S, 10aR)—2,3—dihyro—6—hydroxy—3—(hydroxymethyl)—2—methyl—10H—3,10a—epidithiopyrazino[1,2—a]indole —1,4—dione were added, and stirred at room temperature for 20 hours 30 minutes. Then, saturated sodium bicarbonate aqueous solution was added, and extraction was carried out using dichloromethane. The organic layer was washed with saturated sodium chloride aqueous solution. After drying by anhydrous sodium sulfate, solvent was evaporated under redued pressure. The residue obtained was purified by silica gel chromatography (100 % dichloromethane), and recrystallized from methanol to obtain the object material as light yellow crystals (29.8 mg, yield: 20 %).

$^1H$—NMR($CDCl_3$) δ :3.26(3H, s), 3.34 (1H, d, J=18.8Hz), 3.48 (1H, dd, J=5.65, 9.80Hz), 4.29 (1H, dd, J=9.80, 12.7Hz), 4.32 (1H, d, J=18.8Hz), 4.48 (1H, dd, J=5.65, 12.7Hz), 7.22 (1H, dd, J=7.50, 7.50Hz), 7.33—7.38 (2H, m), 7.94 (1H, d, J=7.50Hz)
High resolution mass spectrum : $C_{13}N_{13}N_2O_3S_2$ ($MH^+$)
Calcd.:m/z 309.0368
Measured.:m/z 309.0366

Example 4

Synthesis of the compound (6) wherein X is hydroxyl group:(3S, 10aR)—2,3—dihydro—6—hydroxy—3—(hydroxymethyl)— 2—methyl—10H—3,10a—epidithiopyrazino[1,2—a]indole— 1,4—dione

Process 1

Synthesis of (3S, 5aS, 6S, 10aR)—2,3,5a,6—tetrahydro—6— hydroxy—3—(hydroxymethyl)—2—methyl—10H—3,10a—epidithiopyrazino[1,2—a]indole—1,4—dione

Acetylgliotoxin (154 mg, 0.418 mmol) was dissolved in 32 mL ethanol, and 68 mg (0.357 mmol p-toluene sulfonic acid was added. After stirring at 100 °C for 14 hours, the solvent was evaporated under reduced pressure, and the residue was purified by silica gel chromatography (dichloromethane : methanol = 99 : 1) to obtain the object material as light yellow solid. (50.8 mg yield : 37%)

$^1H$—NMR ($CDCl_3$) δ :2.94 (1H, D, J=18.0Hz), 3.1 (3H, s), 3.74 (1H, d, J=18.0Hz), 4.10 (1H, dd, J=5.78, 9.53Hz), 4.23(1H, dd, J=9.53, 12.6Hz), 4.44 (1H, dd, J=5.78, 12.6Hz), 4.81 (2H, s), 5.77 (1H, d, J=9.90Hz), 5.87 (1H, s), 5.90-6.00 (2H, m)
FAB MASS:327 ($MH^+$)

Process 2

Synthesis of (3S, 10aR)—2,3—dihydro—6—hydroxy—3— (hydroxymethyl)—2—methyl—10H—3,10a—epidithiopyrazino [1,2—a]indole—1,4—dione

(3S, 5aS, 6S, 10aR)—2,3,5a,6—tetrahydro—6—hydroxy—3—(hydroxymethyl)—2—methyl—10H—3,10a—epidithiopyrazino [1,2—a]indole—1,4—dione (50.8 mg, 0.156 mmol) was dissolved in 3.5 mL toluene, and 37.9 mg (0.154 mmol) o-chloranil was added. After stirring at 100 °C for 2 hours 30 minutes, 14.2 mg (0.0578 mmol) o-chloranil was further added, and stirred at 100 °C for 1 hour. The solvent was evaporated under reduced pressure, and the residue obtained was purified by silica gel chromatography (dichloromethane: ethyl acetate = 8 : 2) to obtain the object material as light yellow solid (30.9 mg). It was recrystallized from methanol to obtain light yellow crystals (20.1 mg, yield

: 40%)

$^1$H—NMR ($CDCL_3$) δ :3.27(3H, s), 3.31 (1H, d, J=18.6Hz), 3.33 (1H, dd, J=5.70, 10.1Hz), 4.31 (1H, dd, J=10.1, 12.9Hz), 4.33 (1H, d, J=18.6Hz), 4.49 (1H, dd, J=5.70, 12.9Hz), 6.82 (1H, d, J=7.35Hz), 6.90 (1H, d, J=8.40Hz), 7.15 (1H, dd, J=7.35, 8.40Hz), 10.5 (1H, s)
High resolution mass spectra : $C_{13}N_{13}N_2O_4S_2$ ($MH^+$)
Calcd.:m/z 325.0317
Measured:m/z 325.0330

Example 5

Synthesis of the compound (13) wherein X is acetoxy group : (3 S, 10aR)—6—acetoxy—2,3—dihydro—3—(hydroxymethyl)— 2—methyl—10H—3,10a—epidithiopyrazino[1,2—a]indole—1, 4—dione

Process 1

(3S, 5aS, 6S, 10aR)—2,3,5a,6—tetrahydro—6—hydroxy—3— (hydroxymethyl)—2—methyl—10H—3,10a—[epithio—(4— methoxyphenyl)methanothio]pyrazino[1,2-a]indole—1,4— dione

(3S, 5aS, 6S, 10aR)—6—acetoxy—2,3,5a,6—tetrahydro3—(hydroxymethyl)—2—methyl—10H—3,10a—[epithio— (4—methoxyphenyl)methanothio]pyrazino[1,2—a]indole—1,4—dione (489 mg, 1.00 mmol) was dissolved in 85 mL ethanol, and 4.2 mL (16.8 mmol) 4N HCl dioxane solution and 4.2 mL water were added. The mixture was refluxed for 1 hour, and stirred at 80 °C for 13 hours 45 minutes. Then, 4.2 mL (16.8 mmol) 4N HCl dioxane solution was added, and refluxed for 10 hours 30 minutes. The solvent was evaporated under reduced pressure, and to the residue, saturated sodium bicarbonate aqueous solution was added. Extraction was carried out by using dichloromethane, and the organic layer was washed with saturated sodium chloride aqueous solution. After drying by anhydrous sodium sulfate, the solvent was evaporated under reduced pressure, and the residue obtained was purified by silica gel chromatography (100 % dichloromethane) to obtain the object material as light yellow solid (356 mg, yield : 80%)

$^1$H—NMR ($CDCL_3$) δ :2.90 (1H, d, J=16.8Hz), 3.21 (3H, s), 3.33 (1H, d, J=16.8Hz), 3.39—3.51 (1H, m), 3.79 (3H, s), 3.84 (1H, dd, J=9.45, 12.7Hz), 4.64 (1H, dd, J=5.70, 12.7Hz), 4.88 (1H, d, J=12.6Hz), 4.95 (1H, d, J=12.6Hz), 5.25 (1H, s), 5.76-5.81 (1H, m), 5.90—5.95 (2H, m), 6.21 (1H, s), 6.85 (2H, d, J=8.70Hz), 7.33 (2H, d, J=8.70Hz)
ESI MASS:447.1 ($MH^+$)

Process 2

Synthesis of (3S, 10aR)—2,3—dihydro—6—hydroxy—3— (hydroxymethyl)—2—methyl—10H—3,10a—[epithio—(4— metoxyphenyl)methanothio]pyrazino[1,2—a]indole—1,4— dione

(3S, 5aS, 6S, 10aR)—2,3,5a,6—tetrahydro—6—hydroxy—3—(hydroxymethyl)—2—methyl—10H—3,10a— [epithio—(4—methoxyphenyl)methanothio]pyrazino[1,2—a]indole—1,4—dione (410 mg, 0.918 mmol) was dissolved in 70 mL toluene, and 238 mg (0.968 mmol) o-chloranil was added. After heating at 100 °C for 2 hours 30 minutes, the solvent was evaporated under reduced pressure, and the residue obtained was purified by silica gel chromatography (100 % dichloromethan) to obtain 379 mg yellow oil. In soluble matters in methanol were filtered, and the object material was obtained as light yellow solid. (214 mg, yield 53%)

$^1$H—NMR ($CDCL_3$) δ :2.73 (1H, dd, J=5.18, 10.1Hz), 3.22 (1H, d, J=17.7Hz), 3.27 (3H, s), 3.78 (3H, s), 3.91 (1H, dd, J=10.1, 12.9Hz), 3.95 (1H, d, J=17.7Hz), 4.69 (1H, dd, J=5.18, 12.9Hz), 5.33 (1H, s), 6.80 (1H, d, J=8.10Hz), 6.83 (2H, d, J=8.70Hz), 6.93 (2H, d, J=8.10Hz), 7.17 (1H, dd, J=8.10, 8.10Hz), 7.32 (2H, d, J=8.70Hz), 10.7 (1H, s)
ESI MASS:445.2 ($MH^+$)

Process 3

Synthesis of (3S, 10aR)—6—acetoxy—2,3—dihydro—3— (hydroxymethyl)—2—methyl—10H—3,10a—[epithio—(4— methoxyphenyl)methanothio]pyrazino[1,2—a]indole—1,4— dione

(3S, 10aR)—2,3—dihydro—6—hydroxy—3—(hydroxymethyl)—2—methyl—10H—3,10a—[epithio—(4— methoxyphenyl) methanothio]pyrazino[1,2—a]indole—1,4—dione (78 mg, 0.175 mmol) was dissolved in a solvent mixture of 12 mL acetonitrile and 9.5 mL dichloromethane, and 2.28 g (16.5 mmol) potassium carbonate was added. After stirring at room temperature for 10 minutes, 16.5 mL (0.175 mmol) glacial acetic acid was added, and further stirred at room temperature for 4 hours. Potassium carbonate was filtered, and the solvent was evaporated under reduced. The residue obtained was purified by silica gel chromatography (dichloromethane : methanol = 95 : 5), and further purified by preparative silica gel plate (dichloromethane : methanol = 25 : 1, developed twice) to obtain the object material. 26.8 mg (0.0507 mmol, yield : 29%)

$^1$H—NMR ($CDCL_3$) δ :2.39 (3H, s), 2.71 (1H, dd, J=4.58, 10.5Hz), 3.24 (3H, s), 3.32 (1H, d, J=17.9Hz), 3.77 (3H, s), 3.83 (1H, dd, J=10.5, 12.8Hz), 4.06 (1H, d, J=17.9Hz), 4.57 (1H, dd, J=4.58, 12.8Hz), 6.07 (1H, s), 6.82 (2H, d, J=8.85Hz), 7.07 (1H, d, J=8.10Hz), 7.20 (1H, d, J=6.60Hz), 7.26 (1H, dd, J=6.60, 8.10Hz), 7.40 (2H, d, J=8.85Hz)
ESI MASS : 509.2 ($M^+$+$Na^+$)

Process 4

Synthesis of (32, 10aR)—6—acetoxy—2,3—dihydro—3— (hydroxymethyl)—2—methyl—10H—3,10a—epidithiopyrazino [1,2—a]indole—1,4—dione

(3S, 10aR)—6—acetoxy—2,3—dihydro—3—(hydroxymethyl)—2—methyl—10H—3,10a—[epithio—(4—methoxyphenyl) methanothio]pyrazino[1,2—a]indole—1,4—dione (82 mg, 0.0169 mmol) was dissolved in 12 mL dichloromethane, and 35.2 mg (0.204 mmol) m-chloroperbenzoic acid was added while cooling in an ice bath. After stirring for 10 minutes, 19.4 mg (0.112 mmol) m-chloroperbenzoic acid was further added, and stirred for 25 minutes. 48 mL (0.654 mmol) dimethylsulfide and 75 mL perchloric acid-methanol solution (1/5) were added, and stirred at room temperature for 1 hour 30 minutes. 25 mL perchloric acid-methanol solution (1/5) was further added, and stirred at room temperature for 2 hours. Saturated sodium bicarbonate aqueous solution was added, and extraction was carried out using dichloromethan. The organic layer was washed with saturated sodium chloride aqueous solution, and dried by anhydrous sodium sulfate. The solvent was evaporated under reduced pressure. The residue obtained was purified by preparative silica gel plate (dichloromethane : methanol = 25 : 1, developed twice), and recrystallized from methanol to obtain the object material as light yellow crystals.
(24.5 mg, yield : 40%)

$^1$H—NMR ($CDCl_3$ ) δ :2.34 (3H, s), 3.25 (3H, s), 3.33 (1H, d, J=18.6Hz), 3.40 (1H, dd, J=5.63, 9.98Hz), 4.26 (1H, dd, J=9.98, 12.8Hz), 4.39 (1H, d, J=18.6Hz), 4.40 (1H, dd, J=5.63, 12.8Hz), 7.00-7.05 (1H, m), 7.21—7.27 (2H, m)
High resolution mass spectra : $C_{15}H_{15}N_2O_5S_2$ ($MH^+$)
Calcd.:m/z 367.0422
Measured:m/z 367.0417

Example 6

Process 1

Synthesis of the compound wherein X is cyanomethyloxy group : (3S, 10aR)—6—(cyanomethoxy)—2,3—dihydro—3—(hydroxymethyl)—2—methyl—10H-3,10a—a— epidithiopyrazino[1,2—a]indole—1,4—dione

Synthesis of (3S, 10aR)—6—(cyanomethoxy)—2,3—dihydro—3 —(hydroxymethyl)—2—methyl—10H—3,10a[epithio—(4— methoxyphenyl)methanothio]pyrazino[1,2—a]indole—1,4— dione (20)

14 (74.2 mg, 0.167 mmol) was dissolved in 10 mL dimethylformamide, and 3.13 g (22.6 mmol) potassium carbonate and 14 µL (0.201 mmol) bromoacetonitrile were added. After stirring at room temperature for 85 hours 30 minutes, potassium carbonate was filtered, and the solvent was evaporated under reduced pressure. The residue obtained was purified by preparative silica gel plate (dichloromethane : methanol = 25 : 1, developed twice) to obtain 20 as yellow solid. (14.4 mg, 18%)

$^1$H—NMR ($CDCL_3$) δ : 2.88 (1H, dd, J=3.00, 7.05Hz), 3.26 (3H, s), 3.26 (1H, d, J=17.7Hz), 3.77 (3H, s), 3.86 (1H, dd, J=7.05, 13.1Hz), 4.04 (1H, d, J=17.7Hz), 4.59 (1H, dd, J=3.00, 13.1Hz), 4.89 (1H, d, J=16.1Hz), 5.08 (1H, d, J=16.1Hz), 5.44 (1H, s), 6.82 (2H, d, J=8.85Hz), 7.12 (1H, d, J=7.20Hz), 7.21 (1H, d, J=7.80Hz), 7.29 (1H, dd, J=7.20, 7.80Hz), 7.32 (2H, d, J=8.85Hz), ESI MASS, 506.3 (M+$Na^+$)

Process 2

20 (10.5 mg, 0.0217 mmol) was dissolved in 1 mL dichloromethane, and 4.88 mg (0.0283 mmol) m-chloroperbenzoic acid was added while cooling in an ice bath. After stirring for 25 minutes, 2.92 mg (0.0169 mmol) m-chloroperbenzoic acid was further added, and stirred for 20 minutes. 6.6 µL (0.0899 mmol) dimethylsulfide and 13 µL perchloric acid-methanol solution (1/5) were added, and stirred at room temperature for 3 hours. Saturated sodium bicarbonate aqueous solution was added, and extraction was carried out using dichloromethane. The organic layer was washed with saturated sodium chloride aqueous solution, and dried by anhydrous sodium sulfate. The solvent was evaporated under reduced pressure, and the residue obtained was purified by preparative silica gel plate (dichloromethane : methanol = 25 : 1, developed twice) to obtain 21 as light yellow solid. (4.86 mg, 62%)

> $^1$H—NMR ($CDCl_3$) δ 3.27 (3H, s), 3.32 (1H, d, J=18.5Hz), 3.49 (1H, dd, J=5.63, 9.68Hz), 4.30 (1H, dd, J=9.68, 12.8Hz), 4.39 (1H, d, J=18.5Hz), 4.45 (1H, dd, J=5.63, 12.8Hz), 4.76 (2H, s), 7.16-7.25 (3H, m);
> High resolution mass spectra,
> Calcd.:
> $C_{15}H_{14}N_3O_4S_2$ ($M^+$+H) m/z 364.0426
> Measured:m/z 364.0421

Example 7

Process 1

Synthesis of the compound wherein X is methoxycarbonyl methyloxy group : (3S, 10aR)—2,3—dihydro—3—(hydroxymethyl)—6—[(methoxycarbonyl)methyloxy]—2—methyl—10H—3,10a—epidiopyrazino[1,2—a]indole—1,4—dione

Synthesis of (3S, 10aR)—2,3—dihydro—3—(hydroxymethyl)— 6—[(methoxycarbonyl)methoxy]—2—methyl—10H—3,10a— [epithio—(4—methoxyphenyl)methanothio]pyrazino[1,2—a] indole—1,4—dione (23)

14 (126 mg, 0.283 mmol) was dissolved in 4 mL dimethylformamide, and 2.12 g (15.3 mmol) potassium carbonate and 31 µL (0.327 mmol) bromomethyl acetate were added. After stirring at room temperature for 23 hours 30 minutes, 10 µL (0.106 mmol) bromomethyl acetate was further added, and then, stirred at room temperature for 13 hours 30 minutes. Potassium carbonate was filtered, and the solvent was evaporated under reduced pressure. The residue obtained was purified by preparative silica gel plate (hexane : ethyl acetate = 1 : 1, developed three times) to obtain 23 as yellow solid. (116 mg, 80%)

> $^1$H—NMR ($CDCl_3$) δ 3.00 (1H, dd, J=4.65, 10.8Hz), 3.23 (1H, d, J=17.1Hz), 3.26 (3H, s), 3.77 (3H, s), 3.81 (3H, s), 3.85 (1H, dd, J=10.8, 12.6Hz), 4.03 (1H, d, J=17.1Hz), 4.58 (1H, dd, J=4.65, 12.6Hz), 4.85 (1H, d, J=16.5Hz), 4.95 (1H, d, J=16.5Hz), 5.71 (1H, s), 6.81 (2H, d, J=8.70Hz), 6.91 (1H, d, J=8.70Hz), 6.98 (1H, d, J=7.20Hz), 7.20 (1h, dd, J=7.20, 8.70Hz), 7.38 (2H, d, J=8.70Hz), ESI MASS, 517.3 ($MH^+$)

Process 2

20 (99.5 mg, 0.193 mmol) was dissolved in 5 mL dichloromethane, and 41.0 mg (0.0238 mmol) m-chloroperbenzoic acid was added while cooling in an ice bath. After stirring for 20 minutes, 20.1 mg (0.116 mmol) m-chloroperbenzoic acid was further added, and stirred for 10 minutes. 14.0 mg (0.0811 mmol) m-chloroperbenzoic acid was further added, and stirred for 15minutes. 100 µL (1.36 mmol) dimethylsulfide and 120 µL perchloric acid-methanol solution (1/5) were added, and stirred at room temperature for 1 hour 40 minutes. Saturated sodium bicarbonate aqueous solution was added, and extraction was carried out using dichloromethane. The organic layer was washed with saturated sodium chloride aqueous solution, and dried by anhydrous sodium sulfate. The solvent was evaporated under reduced pressure, and the residue obtained was purified by preparative silica gel plate (dichloromethane : methanol = 25 : 1, developed twice) to obtain 24 as light yellow solid. (32.8 mg, 43%)

> $^1$H—NMR ($CDCl_3$) δ 3.26 (3H, s), 3.29 (1H, d, J=18.6Hz), 3.60 (1H, dd, J=5.48, 10.1Hz), 3.81 (3H, s), 4.28 (1H, dd, J=10.1, 12.8Hz), 4.38 (1H, d, J=18.6Hz), 4.44 (1H, dd, J=5.48, 12.8Hz), 4.65 (1H, d, J=15.9Hz), 4.70 (1H, d, J=15.9Hz), 6.98 (1H, d, J=8.10Hz), 7.04 (1H, d, J=7.65Hz), 7.17 (1H, dd, J=7.65, 8.10Hz);
> High resolution mass spectra, Calcd.: $C_{16}H_{17}N_2O_6S_2$ ($M^+$+H) m/z 397.0528, Measured : m/z 397.0520

Example 8

Evaluation of Cytotoxity

Antitumor activity of the synthesized derivatives against various cancer cells was measured by the MTT method. The cancer cells used were mouse carcinoma of the colon cells colon 26 (Gan Kenkyu-sho, Gan Kenkyu-kai) and mouse leukemia cells P388 (Dai-Nippon Seiyaku), and the culture medium contained 10 % fetal calf serum. Each cells were placed in each well of 96 well microplate n an amount of 5 × 103/50 μL/well and 50 μL of a test compound aqueous solution in each concentration was added. After incubating at 37 °C for 3 days, the number of survival cells was measured by the MTT method, and a dose-reaction curve was made. Thereby, the concentration of each test compound inhibiting multiplication at a rate of 50 % ($IC_{50}$) was calculated. The $IC_{50}$ value of each compound was shown in the table.

| Compound | P388 $IC_{50}$[ng/mL] | colon 26 IC $_{50}$ [ng/mL] |
|---|---|---|
| Compound of Example 3 | 3.2 | 58 |
| Compound of Example 4 | 21 | 110 |
| Compound of Example 5 | 13 | 74 |
| Compound of Example 6 | 5.8 | 70 |
| Compound of Example 7 | - | 82 |

Example 9

Anticancer effect in vivo against carcinoma

(A) Evaluationin vivo using mouse carcinoma of the colon cells colon 26 CDFI mice (female : 4 weks age) were bought, and colon 26 subcultured in the subcutaneous tissue of the mouse was taken out, and cut into small pieces by a scissors. The cancer cells were transplanted into subcutaneous tisue of CDFI mice by a trucker. The day was assigned 0 day, and the tumor was measured by a slide caliper after 7 days. The volume of the tumor was calculated, and divided into groups (each group : n = 4).

The text compound was dissolved in dimethylsulfoxide, and diluted with 5 % Tween 80- saline aqueous solution. 0.2 mL of the diluted solution was administrated by intravenous injection or subcutaneous injection. The administration was carried out after 7 days, after 11 days and after 15 days or after 7 days and after 8 days from the transplantation at a rate of once per one day. 21st day from the transplantation, the volume of tumor was measured and the volume of tumor and the inhibition against tumor multiplication rate (I.R.) were calculated.

The volume of tumor was calculated by the following formula.

$$\text{Tumore Volume } [mm^3] = \text{Minor Axis } [mm]^2 \times \text{Major Axis } [mm] \times (1/2)$$

The inhibition against tumor multiplication rate (I.R.) was calculated by the following formula.

$$\text{I.R. } [\%] = \{1 - (\text{Average Tumor Volume of Administration Group / Average Tumor Volume of Control Group})\} \times 100$$

(B) Evaluation in vivo using human carcinoma of the colon cells

HCT-116 Balb/c nu/nu mice (female : 5 weks age) were bought, and HCT-116 (Dainippon Seiyaku) subcultured in the subcutaneous tissue of the mouse was taken out, and cut into small pieces by a scissors. The cancer cells were transplanted into subcutaneous tissue of CDFI mice by a trucker. The day was assigned 0 day, and the tumor was measured by a slide caliper after 7 days. The volume of the tumor was calculated, and divided into groups (each group : n = 2). The text compound was dissolved in dimethylsulfoxide, and diluted with 5 % Tween 80- saline aqueous solution. The diluted solution was administrated by intravenous injection or subcutaneous injection. The

administration was carried out after 7 days, after 11 days and after 15 days from the transplantation at a rate of once per one day. 21st day from the transplantation, the volume of tumor was measured and the volume of tumor and the inhibition against tumor multiplication rate (I.R.) were calculated by the above formulas.

Anticancer Effect against colon 26

| Compound | Dose and Administration Type | Day of Administration | (21st day) | I.R.Max |
|---|---|---|---|---|
| Adriamycin | 10 [mg/kg]×2 (i.v.) | 7, 14th days | 38.9 | 38.9(21st day) |
| Compound of Example 2 | 2.5[mg/kg]×3 (i.p.) | 7,11,15th days | 19.6 | 44.9(10th day) |
| | 5 [mg/kg] ×3 (i.p.) | 7,11,15th days | 11.8 | 56.8(9th day) |
| | 10 [mg/kg]×2 (i.p.) | 7,8th days | 26.0 | 68.8(10th day) |
| Compound of Example 3 | 2.5[mg/kg] ×3 (i.p.) | 7,11,15th days | 35.2 | 35.3( 8th day) |
| | 5 [mg/kg]×3 (i.p.) | 7,11,15th days | 43.7 | 51.6( 8th day) |
| Compound of Example 4 | 2.5[mg/kg]×3 (i.p.) | 7,11,15th days | 40.1 | 54.5(11th day) |
| | 5 [mg/kg]×3 (i.p.) | 7,11,15th days | 52.9 | 58.9(10th day) |
| | 10 [mg/kg]×2 (i.p.) | 7,8th days | 43.5 | 79.8(10th day) |

Anticancer Effect against HCT 116

| Compound | Dose and Administration Type | Day of Administration | I.R. (21st day) | I.R.Max |
|---|---|---|---|---|
| CDDP | 5 [mg/kg]×3 (i.p.) | 7,11,15th days | 15.6 | 47.5(18th day) |
| Compound of Example 4 | 5 [mg/kg]×3 (i.p.) | 7,11,15th days | 63.8 | 66.5(18th day) |

The gliotoxin derivatives of the invention exhibit excellent effect on carcinoma which is insufficient by conventional medicines, and they are very useful in medical industry.

**Claims**

1. An anticancer agent which comprises a gliotoxin derivative represented by the general formula (I) as an active principle.

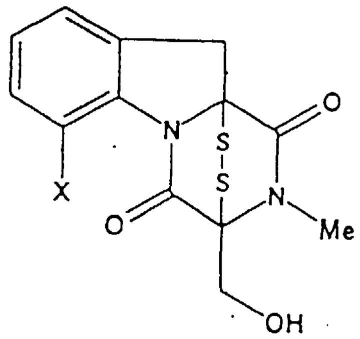

(I)

[In the formula, X represents hydrogen atom, hydroxyl group, an alkoxy group having a number of carbon atoms of 1 to 3, or an acyloxy group having a number of carbon atoms of 2 to 3.]

**2.** The anticancer agent of claim I wherein X is hydrogen atoms, an alkoxy group having a number of carbon atoms of 1 to 3, or an acyloxy group having a number of carbon atoms of 2 to 3.

**3.** The anticancer agent of claim 1 wherein X is hydrogen atoms, hydroxyl group or acetoxy group.

**4.** A gliotoxin derivative represented by the general formula (I)
[In the above formula, X represents an alkoxy group having a number of carbon atoms of 1 to 3, or an acyloxy group having a number of carbon atoms of 2 to 3.]

**5.** The gliotoxin derivative of claim 4 wherein X is hydrogen atom or acetoxy group.

**6.** An anticancer agent which comprises acetylgliotoxin as an active principle.

**7.** A method of producing acetylgliotoxin which comprises culturing a microorganism having acetylgliotoxin-producing ability, and recovering produced acetylgliotoxin.

**8.** The method of producing acetylgliotoxin of claim 6 wherein the microorganism having acetylgliotoxin-producing ability belongs to genus Dichotomomyces.

**9.** The method of producing acetylgliotoxin of claim 7 wherein the microorganism having acetylgliotoxin-producing ability is Dichotomomyces cejpii FERM BP-5738.

**10.** A method of producing acetylgliotoxin which comprises adding an adsorbent to a culture matter of an acetylgliotoxin-producing microorganism to adsorb acetylgliotoxin, and separating the adsorbent from the culture matter.

**11.** A method of producing acetylgliotoxin which comprises adding an acetylgliotoxin soluble organic solvent to a culture matter of an acetylgliotoxin-producing microorganism, and extracting acetylgliotoxin by the organic solvent.

F i g. 1

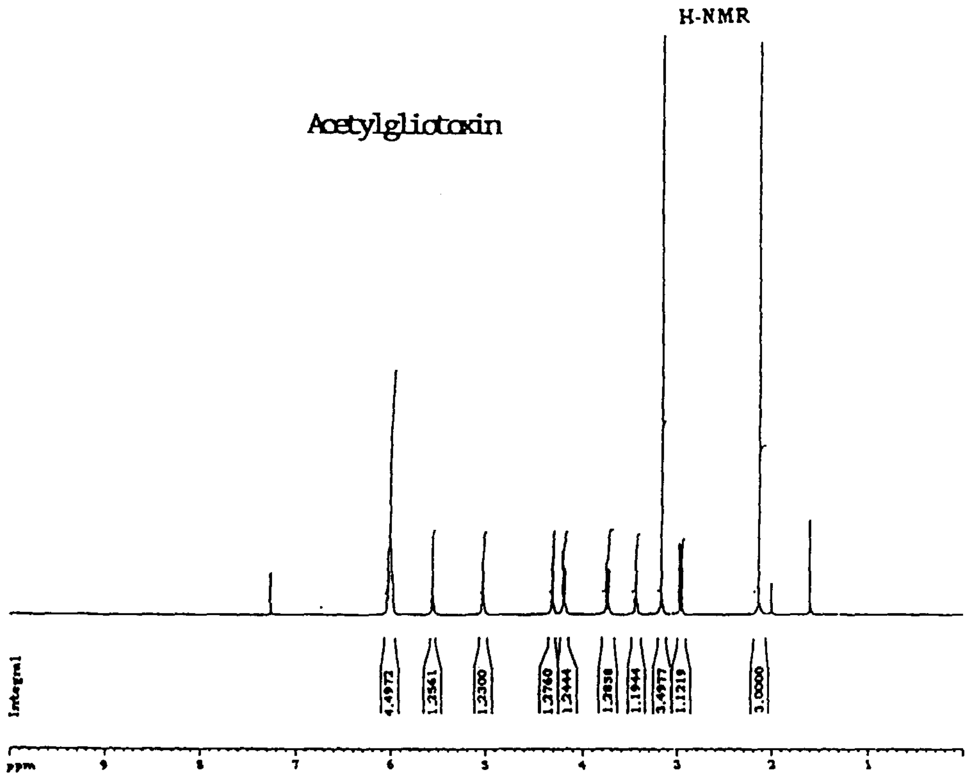
H-NMR
Acetylgliotoxin
Integral
4.4972
1.2561
1.2300
1.2760
1.2444
1.2838
1.1944
3.4977
1.1219
3.0000
ppm 9 8 7 6 5 4 3 2 1

F i g. 2

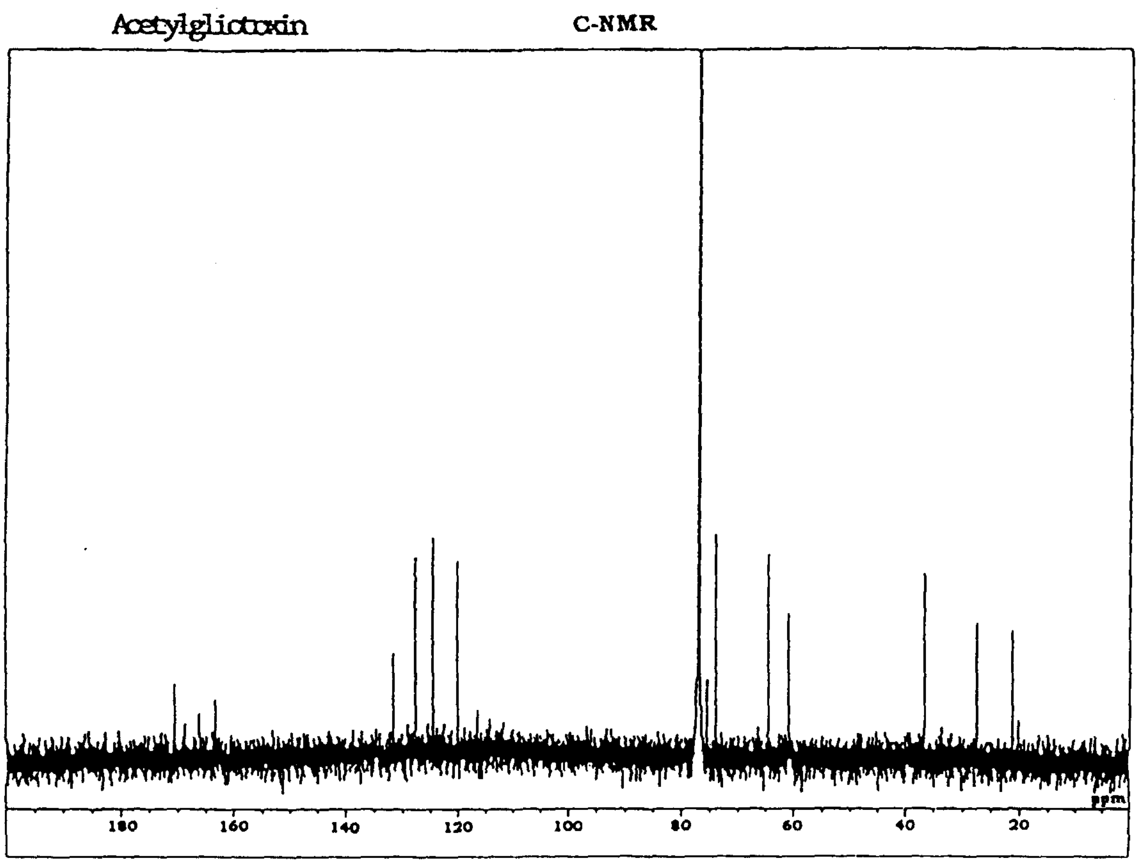
Acetylgliotoxin
C-NMR
180 160 140 120 100 80 60 40 20
ppm

Fig. 3

(A)

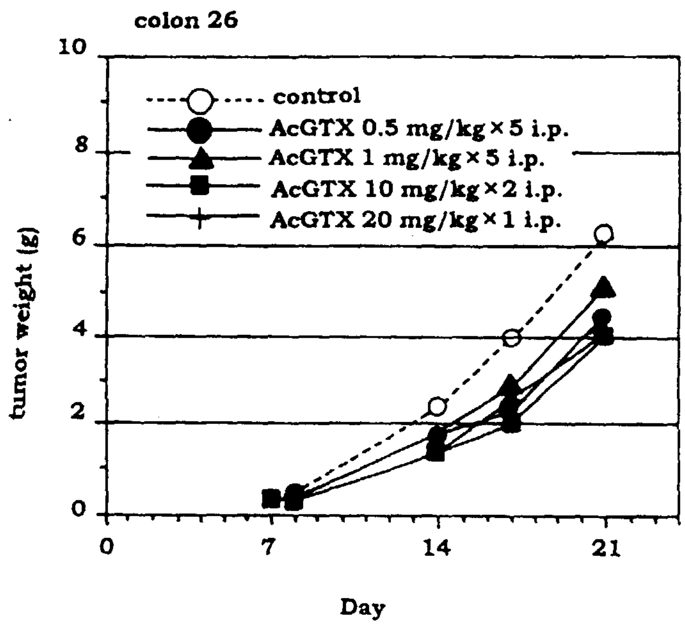
colon 26
control
AcGTX 0.5 mg/kg × 5 i.p.
AcGTX 1 mg/kg × 5 i.p.
AcGTX 10 mg/kg × 2 i.p.
AcGTX 20 mg/kg × 1 i.p.
tumor weight (g)
0
2
4
6
8
10
0
7
14
21
Day

(B)

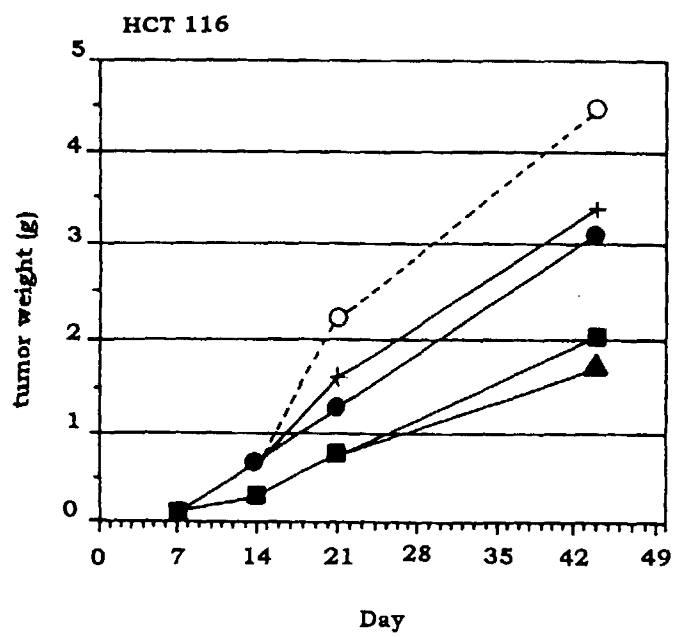
HCT 116
tumor weight (g)
0
1
2
3
4
5
0
7
14
21
28
35
42
49
Day

F i g. 4

(A)

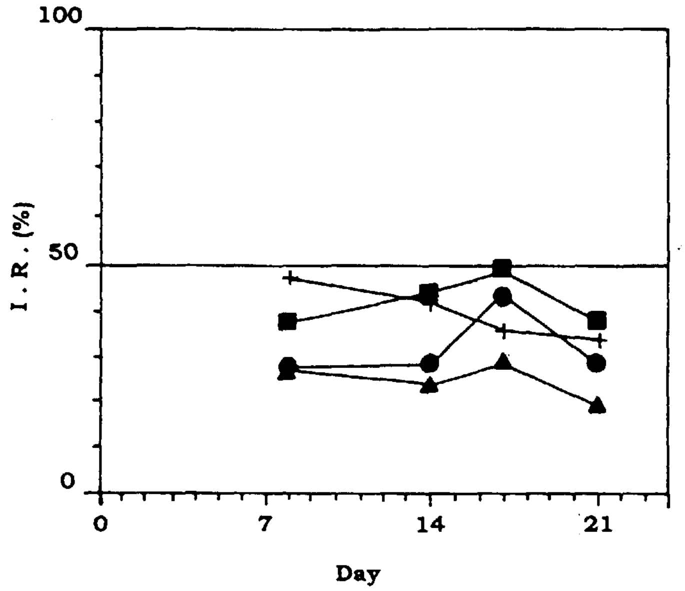


(B)

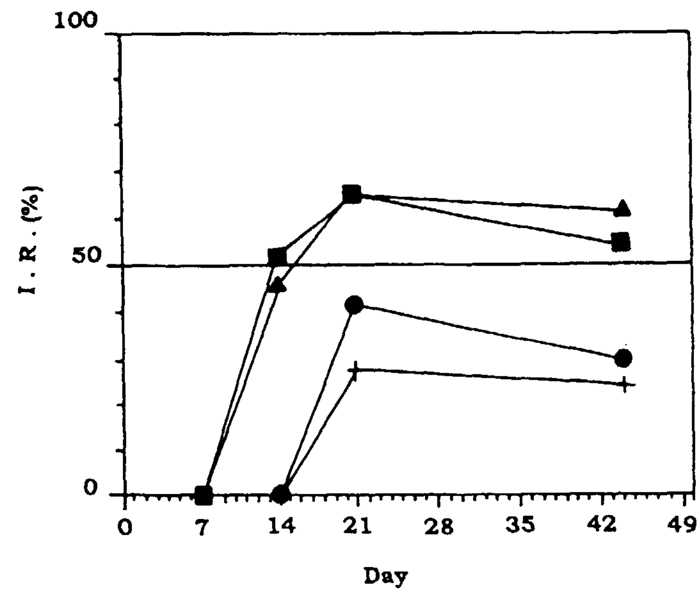

F i g. 5

(A)

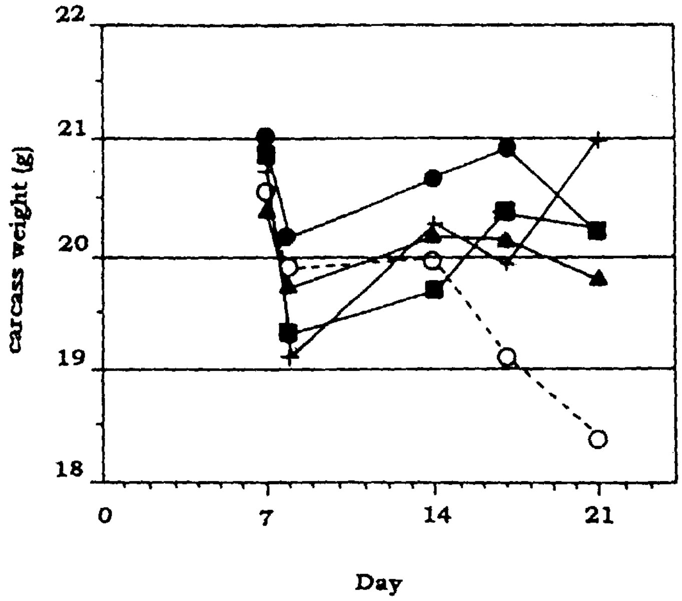
22
21
20
19
18
carcass weight (g)
0
7
14
21
Day

(B)

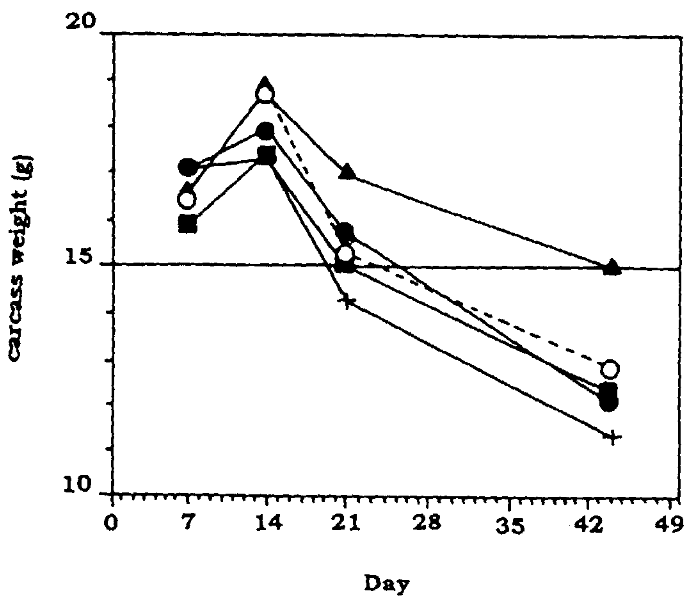
20
15
10
carcass weight (g)
0
7
14
21
28
35
42
49
Day

## INTERNATIONAL SEARCH REPORT

International application No.
PCT/JP97/04404

**A. CLASSIFICATION OF SUBJECT MATTER**

Int. $Cl^6$ C12P17/18, C07D513/18, A61K31/535 // (C12P17/18, C12R1:645)

According to International Patent Classification (IPC) or to both national classification and IPC

**B. FIELDS SEARCHED**

Minimum documentation searched (classification system followed by classification symbols)

Int. $Cl^6$ C12P17/18, C07D513/18, A61K31/535

Documentation searched other than minimum documentation to the extent that such documents are included in the fields searched

Electronic data base consulted during the international search (name of data base and, where practicable, search terms used)

WPI(DIALOG), CA(STN), REGISTRY(CA), BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| X/Y<br>A | The Journal of Antibiotics, Vol. 45, No. 11, p. 1802-1805 (1992) "Inhibition of Transferase by Gliotoxin and Acetylgliotoxin", Didier Van Der Pyl et al. | 7/10-11<br>1-6, 8-9 |
| A | J. Chem. Soc. Perkin Trans. 1, p. 301-304 (1988), "New Co-metabolites of Gliotoxin in Gliocladium virens", Gordon W. Kirby et al. | 1 - 11 |
| A | Phytochemistry, Vol. 32, No. 1, p. 197-198 (1993), "Metabolites of Gliocladium Flavofuscum", Anthony G. Avent et al. | 1 - 11 |
| A | Molecular Immunology, Vol. 23, No. 2, p. 231-235 "Structural Relationship of Epipolythiodioxopiperazines and Their Immunomodulating Activity", Arno M. et al. | 1 - 11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 30, 1998 (30. 01. 98) | February 10, 1998 (10. 02. 98) |
| Name and mailing address of the ISA/<br>Japanese Patent Office | Authorized officer |
| Facsimile No. | Telephone No. |

Form PCT/ISA/210 (second sheet) (July 1992)